# EUROPEAN PATENT SPECIFICATION

(11) **EP 3 763 351 B1**
(45) Date of publication and mention of the grant of the patent: **28.01.2026**
(21) Application number: 19190802.9
(22) Date of filing: 08.08.2019
(51) Int. Cl.: A61K 8/04, A61K 8/31, A61K 8/73, A61Q 5/00

(54) **AEROSOL TEXTURIZING HAIR FILLER WITH FIBERS**
AEROSOLTEXTURIERENDES HAARFÜLL MIT FASERN
AÉROSOL COMPORTANT DES FIBRES PERMETTANT DE REMPLIR LES "TROUS" CAPILLAIRES

(30) Priority: 06.07.2019 US 201962871095 P
(43) Date of publication of application: 13.01.2021
(73) Proprietor: American Spraytech, L.L.C., North Branch, New Jersey 08876-6032 (US)
(72) Inventor: Mustafa, Aaysha, New Jersey, 08902 (US); Ipaye, Rasheedat, New Jersey, 07018 (US); Lalwani, Manav A., New Jersey, 07094 (US); Parikh, Bhoomi, New Jersey, 08830 (US); Gribben, Samantha F., New Jersey, 07008 (US)
(74) Representative: Sandersons

(56) References cited:
- WO-A1-2018/221519
- JP-A- 2010 037 200
- JP-A- 2016 065 030
- JP-A- H11 158 034
- US-A1- 2018 344 589
- US-A1- 2019 008 751
- US-B1- 9 492 363
- DATABASE WPI Week 201017, Derwent World Patents Index; AN 2010-B81444, XP002797474
- DATABASE WPI Week 201883, Derwent World Patents Index; AN 2018-96919Q, XP002797475
- DATABASE WPI Week 201632, Derwent World Patents Index; AN 2016-25475Y, XP002797476

## Description

### FIELD OF THE INVENTION

One or more embodiments of the present invention relate to aerosol-sprayable texturizing hair colorant compositions containing fibers, and dispensers therefor.

### BACKGROUND OF THE INVENTION

The desire to cover bald spots on the scalp and to fill in areas of the scalp where hair is thin, to give the appearance of fuller hair, has given rise to a multitude of ingredient technologies and treatments. There are also products that are intended to provide some color coverage of hair roots. However, current products can be difficult to use and hard to remove, frequent use of which may result in damage and loss of hair. Hair enhancements and fillers can be difficult to apply evenly for a natural appearance.

Aerosol spray devices are often used to apply products to the scalp and hair. However, these spray devices can become clogged if the product contains particulates and/or fibers. US9492363 discloses a sprayable color hair product.

There has been, and remains, a need for products and treatment regimen that creates the appearance of fuller hair by covering bald spots and areas on the scalp where hair is thin, while at the same time being easily and reliably applied in an even and controlled pattern. Products that also provide colorant to the hair would be beneficial.

### BRIEF DESCRIPTION OF THE DRAWINGS

For a full understanding of the apparatus and methods of the present disclosure reference should be made to the following detailed description and the accompanying drawings, wherein:
FIG. 1 is a side sectional view of an embodiment of the actuator.
FIG. 2 is a sectional view of an embodiment of the orifice insert.
FIG. 3 is a side sectional view of an embodiment of the actuator.
FIG. 4 is an exploded diagram of an embodiment of the valve system.
FIG. 5 is a side sectional view of an embodiment of the valve system.
FIG. 6 is a side view of an embodiment of the can.

### SUMMARY OF THE INVENTION

Embodiments of the invention provide compositions and methods that create the appearance of fuller hair when applied to the scalp by covering bald spots and creating texture on areas of the scalp where hair is thin. Advantageously, compositions of the present invention may be easily and reliably applied in an even and controlled pattern.

An aerosol texturizing colorant composition comprising:
(a) a hair filler composition that comprises:
   from about 0.1 to about 20 wt. % of cellulose fibers, based upon the total weight of the hair filler composition, wherein the average length of the cellulose fibers is from about 20 to about 25 microns;
   a texturizing agent;
   a conditioning agent; and
   a carrier comprising as a film forming polymer a trimethylsiloxysilicate resin, one or more colorants, and a non-aqueous solvent;
   wherein the texturizing agent is a hollow microsphere complex selected from the group consisting of Silica (and) Ethylene/Methacrylate Copolymer (and) Isopropyl Titanium Triisostearate, Mica (and) Ethylene/Methacrylate Copolymer (and) Isopropyl Titanium Triisostearate, Talc (and) Ethylene/Methacrylate Copolymer (and) Isopropyl Titanium Triisostearate, Iron Oxides (and) Ethylene/Methacrylate Copolymer (and) Isopropyl Titanium Triisostearate, and combinations thereof and wherein the amount of texturing agent is from about 0.1 to about 10 wt. %, based upon the total weight of the hair filler composition; and
(b) a propellant; wherein the aerosol composition is dispensable by an aerosol dispensing system for a time period of from 5-10 seconds, every 24 hours for at least 14 days, without the dispensing system becoming clogged.
invention further provides a method for creating the appearance of fuller hair by covering bald spots and areas on the scalp where hair is thin, comprising: evenly applying the aerosol sprayable colorant composition of to the scalp in a controlled pattern.

### DETAILED DESCRIPTION OF ILLUSTRATIVE EMBODIMENTS

In embodiments disclosed throughout this specification, the aerosol cosmetic composition includes a hair filler composition and a propellant. The hair filler composition may be a liquid suspension at standard conditions of temperature and pressure, and includes fibers, a texturing agent, a conditioner, and a carrier.

### Fibers

The fibers are cellulose-based fibers. Examples of cellulose-based fibers that are commercially available include those marketed under the tradename Cell-u-lash 40 by Kobo Products, Inc.

In embodiments disclosed throughout this specification, the amount of fibers may be at least 0.1, in other embodiments, at least 0.2, in other embodiments, at least 0.5, in other embodiments, at least 1, in other embodiments, at least 1.5, in other embodiments, at least 2, in other embodiments, at least 2.5, in other embodiments, at least 3, in other embodiments, at least 3.5, in other embodiments, at least 4, in other embodiments, at least 4.5, in other embodiments, at least 5, in other embodiments, at least 5.5, in other embodiments, at least 6, in other embodiments, at least 6.5, in other embodiments, at least 7, in other embodiments, at least 7.5, in other embodiments, at least 8, in other embodiments, at least 8.5, in other embodiments, at least 9, in other embodiments, at least 9.5, in other embodiments, at least 10, in other embodiments, at least 10.5, and in other embodiments, 11 wt. %, based upon the total weight of the hair filler composition.

In embodiments disclosed throughout this specification, the amount of fibers may be no more than 20 wt. %, in other embodiments, no more than 15 wt. %, in other embodiments, no more than 12 wt. %, and in other embodiments, no more than 10 wt. %, based upon the total weight of the hair filler composition.

In embodiments disclosed throughout this specification, the amount of fibers is from about 1 to about 20 wt. %, in other embodiments, from about 2 to about 15 wt. %, and in other embodiments, from about 5 to about 10 wt. %, based upon the total weight of the hair filler composition.

In embodiments disclosed throughout this specification, the amount of fibers may be at least 0.01, in other embodiments, at least 0.02, in other embodiments, at least 0.05, in other embodiments, at least 0.1, in other embodiments, at least 0.2, in other embodiments, at least 0.3, in other embodiments, at least 0.4, in other embodiments, at least 0.5, in other embodiments, at least 0.6, in other embodiments, at least 0.7, in other embodiments, at least 0.8, in other embodiments, at least 0.9, in other embodiments, at least 1.0, in other embodiments, at least 1.5, and in other embodiments, 2 wt. %, based upon the total weight of the aerosol cosmetic composition.

In embodiments disclosed throughout this specification, the amount of fibers may be no more than 2 wt. %, in other embodiments, no more than 1.5 wt. %, in other embodiments, no more than 1.2 wt. %, and in other embodiments, no more than 1 wt. %, based upon the total weight of the hair filler composition.

In embodiments disclosed throughout this specification, the amount of fibers may be from about 0.05 to about 2 wt. %, in other embodiments, from about 0.1 to about 1.5 wt. %, and in other embodiments, from about 0.25 to about 1 wt. %, based upon the total weight of the aerosol cosmetic composition.

### Texturing Agent

In embodiments disclosed throughout this specification, the composition includes at least one texturing agent. The texturing agent is a hollow microsphere complex, such as those identified by the INCI Names: Silica (and) Ethylene/Methacrylate Copolymer (and) Isopropyl Titanium Triisostearate, Mica (and) Ethylene/Methacrylate Copolymer (and) Isopropyl Titanium Triisostearate, Talc (and) Ethylene/Methacrylate Copolymer (and) Isopropyl Titanium Triisostearate, or Iron Oxides (and) Ethylene/Methacrylate Copolymer (and) Isopropyl Titanium Triisostearate. Suitable starches include rice, tapioca, and corn starch.

In embodiments disclosed throughout this specification, the amount of texturing agent may be at least 0.1, in other embodiments, at least 0.2, in other embodiments, at least 0.5, in other embodiments, at least 0.7, in other embodiments, at least 1, in other embodiments, at least 1.2, and in other embodiments, at least 1.5 wt. %, based upon the total weight of the aerosol cosmetic composition.

The upper limit of the amount of texturing agent may be at most 10 %, in other embodiments, at most 5 %, and in other embodiments, at most 2.5 %, based upon the total weight of the aerosol cosmetic composition.

### Conditioning Agent

In embodiments disclosed throughout this specification, the composition includes at least one hair or skin conditioner. Examples of conditioning agents include starches and modified starches, such as rice, tapioca, and corn starches, and aluminum salts of modified starches. In embodiments disclosed throughout this specification, the starch may be aluminum starch octenylsuccinate.

In embodiments disclosed throughout this specification, the amount of conditioning agent may be at least 0.1, in other embodiments, at least 0.2, in other embodiments, at least 0.5, in other embodiments, at least 0.7, in other embodiments, at least 1, in other embodiments, at least 1.2, and in other embodiments, at least 1.5 wt. %, based upon the total weight of the aerosol cosmetic composition.

In embodiments disclosed throughout this specification, the upper limit of the amount of conditioning agent is not limited, with the proviso that the overall solid load of the composition is no more than 45 %. In embodiments disclosed throughout this specification, the amount of conditioning agent may be at most 10 %, in other embodiments, at most 5 %, and in other embodiments, at most 2.5 %, based upon the total weight of the aerosol cosmetic composition.

### Carrier

In embodiments disclosed throughout this specification, the carrier includes a film forming polymer, one or more cosmetic colorants, and a solvent.

### Film Former

The film forming polymer includes a trimethylsiloxysilicate resin. It may be oil soluble. In embodiments disclosed throughout this specification, the film forming polymer advantageously binds the fibers to the skin or hair.

Suitable film forming polymers may include low molecular weight non-polar thermoplastic polyolefins, oil soluble silicone polymers, co-polymers, terpolymers, and cross polymers thereof.

Suitable non-polar thermoplastic polyolefins include polymers and copolymers prepared from monomers including paraffin (sometimes also referred to as alkane), olefin, and diolefin monomers. Suitable non-polar thermoplastic polyolefins also include hydrogenated polyolefins such as hydrogenated polycyclopentadienes, hydrogenated polyolefins. Examples of suitable monomers include piperylene, butane, butene, pentane, pentene, and cyclopentadiene.

Examples of film formers include hydrogenated polycyclopentadiene, trimethylsiloxysilicate, cyclomethicone and dimethicone crosspolymers, homopolymer of isoprene, hydrogenated homopolymer of isoprene, and trimethylsiloxysilicate/dimethiconol crosspolymer, polyvinylpyrrolidone/vinyl acetate, acrylates, acrylate/silicone copolymers, polybutene, polyethylene, polyurethane-14, AMP-acrylates copolymer, acralates/octylacrylamide copolymer, polyquaternium-11, terpolymer of vinylcaprolactum/vinyl pyrrolidone and methylacrylamide.

In embodiments disclosed throughout this specification, the oil soluble film forming polymer is selected from silicone resins, such as siloxysilicates. Silicone resin nomenclature is known in the art as "MDTQ" nomenclature, whereby a silicone resin is described according to the various monomeric siloxane units which make up the polymer. In embodiments disclosed throughout this specification, the silicone resin may be a MQ type of silicone resin. The letter M denotes the monofunctional unit (CH3)₃SiO_{1/2}. This unit is considered to be monofunctional because the silicone atom only shares one oxygen when the unit is part of a polymer. Similarly, the symbol Q denotes the tetrafunctional unit, SiO_{4/2} wherein all four oxygens bonded to the silicone atom are bonded to the rest of the polymer. MQ silicone resins are sometimes referred to as siloxysilicates. A non-limiting example of a siloxysilicate is trimethylsiloxysilicate, which may be represented by the following formula: [(CH₃)₃-Si-O]ₓ-(SiO_{4/2})_{y} (i.e, MQ units) wherein x and y may, for example, range from 50 to 80.

Silicone crosspolymers are sometimes referred to as silicone elastomers, and are based upon crosslinked dimethicone. Silicone crosspolymer gels are often provided as blends of silicone elastomer in silicone fluid, which acts as a diluent. Examples of the silicone fluid diluent include dimethicone and cyclopentasiloxane. Examples of silicone crosspolymers include cyclomethicone and dimethicone crosspolymers.

In embodiments disclosed throughout this specification, the film-forming polymer may be pre-mixed with a solubilizer, such as methyl trimethicone. Other solubilizers include ethyl trisiloxane, cyclomethicone and dimethicone.

Examples of trimethylsiloxysilicate resins that are premixed with solubilizers are designated with the INCI names trimethylsiloxysilicate (and) dimethicone, trimethylsiloxysilicate (and) cyclomethicone, trimethylsiloxysilicate (and) methyl trimethicone, and trimethylsiloxysilicate (and) ethyl trisiloxane.

Oil soluble film forming polymers suitable for the compositions of the present invention are commercially available from several sources. Commercially available oil soluble film formers include those sold under the trade name Koboguard^{®}, such as the Koboguard 5400 series of products, and the Koboguard MQ resins such as Koboguard MQ65TMF (available from Kobo Products), those available under the trade names RTM 2-9040 (available from Dow Corp), those available under the trade names SS4230, SS4267, SR1000 (available from Momentive), those available under the trade names LIR-30, LIR-50, LIR200, LIR290 (available from Kurray Co.), and those available under the trade name TMS 803 (available from Wacker Chemie AG). Additional film formers are commercially available, for example, as polyvinylpyrrolidone/vinyl acetate as Luvisikol VA, by BASF Corp., polyurethane-14 and AMP-acrylates copolymer as DynamX, by Akzonobel, Octylacrylamide/ Acrylates/Butylaminoethyl Methacrylate copolymer as Amphomer LV71 by Akzonobel, polyquaternium-11 as Gafquat 755N by ISP, and terpolymer of vinylcaprolactum/vinyl pyrrolidone and methylacrylamide as Aquaflex SF-40 by ISP.

In embodiments disclosed throughout this specification, the oil soluble film forming polymer includes trimethylsiloxysilicate. In embodiments disclosed throughout this specification, the trimethylsiloxysilicate (TMS) resin may be commercially available from Momentive Performance Materials under the tradename SR1000 or from Wacker Chemie AG under the tradename TMS 803.

In embodiments disclosed throughout this specification, the hair filler composition includes from about 1 wt.% to about 20 wt.% of film forming polymer, in other embodiments from about 1.5 to about 15 wt.%, and in other embodiments, from about 2 to about 10 wt. %, based upon the total weight of the hair filler composition.

In embodiments disclosed throughout this specification, where the film forming polymer is pre-mixed with a solubilizer, the hair filler composition may include from about 3 wt.% to about 40 wt.% of the pre-mix, in other embodiments from about 4 to about 35 wt.%, in other embodiments from about 5 to about 30 wt.%, and in other embodiments from about 6 to about 25 wt.%, based upon the total weight of the hair filler composition.

In embodiments disclosed throughout this specification, the amount of film forming polymer may be from about 0.2 wt.% to about 0.6 wt.%, in other embodiments from about 0.25 to about 0.5 wt.%, based upon the total weight of the aerosol cosmetic composition.

### Cosmetic Colorant

In embodiments disclosed throughout this specification, the aerosol spray cosmetic composition includes a cosmetic colorant. Cosmetic colorants can be used individually or as mixtures to achieve the desired colors.

Examples of cosmetic hair colorants include FD&C aluminum lakes, FD&C calcium lakes, D&C blues, D&C browns, D&C yellows, D&C oranges, D&C reds, D&C greens, D&C ultra marines, FD&C blues, FD&C browns, FD&C yellows, FD&C oranges, FD&C reds, FD&C greens, FD&C ultra marines, black iron oxide, aluminum powder, bronze powder, chromium, chromium hydroxide, iron oxides, titanium dioxide, zinc oxide, zinc myristate, mica, titanated mica, talc, corn starch, aluminum starch, pearlizing agents, silica, silica silyate, spherical silica, fumed silica, aluminum silicate calcium silicate, silk powder, and polyester glitter flakes. In one or more embodiments the cosmetic colorant can be surface-treated with silicones, silanes, lecithin, cationic polymer, N-lauroyl-L-lysine, amino acids, triethoxycaprylylsilane, or some combination thereof.

In embodiments disclosed throughout this specification, the cosmetic colorant includes one or more of titanium dioxide (C.I. 77891), yellow iron oxide (C.I. 77492), red iron oxide (C.I. 77491), black iron oxide (C.I. 77499), and mixtures thereof. Yellow iron oxide is sometimes referred to as Pigment Yellow 42. Iron oxides, depending upon the degree of hydration, are sometimes referred to as Iron(III) oxide-hydroxides.

The total amount of the cosmetic colorant may be stated based upon the hair filler composition, and may also be stated based upon the total aerosol cosmetic composition, i.e.including the propellant.

In embodiments disclosed throughout this specification, the at least one cosmetic colorant may be present in the hair filler composition in an amount of from about 0.4 wt.% to about 25 wt.%, in other embodiments, from about 0.4 wt. % to about 22 wt. %, based upon the total weight of the hair filler composition.

n embodiments disclosed throughout this specification, the at least one cosmetic colorant may be present in the aerosol cosmetic composition in an amount of from about 0.01 wt.% to about 1.2 wt.%, in other embodiments, from about 0.02 wt. % to about 1.1 wt. %, based upon the total weight of the aerosol cosmetic composition.

Advantageously, in embodiments disclosed throughout this specification, the total amount of cosmetic colorant may be reduced, compared to hair colorant or root concealer compositions that do not contain fibers. In embodiments disclosed throughout this specification, the reduction in colorant leads to enhanced sprayability of the compositions, while good color coverage is maintained.

### Solvent

In embodiments disclosed throughout this specification, the aerosol spray cosmetic composition may include one or more hydrophobic solvents. The solvents may function as a diluent, to increase wetting, to improve spreading, to adjust particle size and/or to influence film thickness.

Suitable solvents include those that readily solubilize the film forming polymer. Additionally, the solvent should be able to readily form a dispersion with the fibers and pigments.

Examples of suitable solvents include volatile silicones, which may be cyclic or non-cyclic. In embodiments disclosed throughout this specification, the solvent may be characterized by having a heat of vaporization (kJ/kg) at 25 °C of from about 100 to about 300 kJ/kg, in other embodiments, from about 120 to about 200 kJ/kg.

In embodiments disclosed throughout this specification, the volatile silicone solvent may be a low viscosity silicone fluid, characterized by a viscosity (measured at 25 °^{C}) of from about 0.65 mm²/s (cSt) to about 20 mm²/s (cSt), and in other embodiments, characterized by a viscosity of less than about 5 mm²/s (cSt) at 25 °C.

In embodiments disclosed throughout this specification, the volatile silicone solvent may be a polyorganosiloxane having from 2 to 6 silicon atoms. In embodiments disclosed throughout this specification, the volatile silicone solvent may be an aliphatic volatile silicone. In embodiments disclosed throughout this specification, the volatile silicone solvent may be a trisiloxane. In embodiments disclosed throughout this specification, the solvent may be 3-ethylheptamethyl-trisiloxane (INCI name: ethyl trisiloxane).

In embodiments disclosed throughout this specification, the hair filler composition may include one or more solvents in an amount of from about 35 wt.% to about 75 wt.%, in other embodiments, from about 40 wt. % to about 70 wt. %, based upon the total weight of the hair filler composition.

In embodiments disclosed throughout this specification, the aerosol cosmetic composition may include at least one solvent in an amount of from about 3 wt.% to about 10 wt.%, in other embodiments, from about 4 wt. % to about 7 wt. %, based upon the total weight of the aerosol cosmetic composition.

In embodiments disclosed throughout this specification, the amount of water in the composition may be limited. In embodiments disclosed throughout this specification, the amount of water in the hair filler composition may be from zero to about 10 wt. %, based upon the total weight of the hair filler composition. In embodiments disclosed throughout this specification, the amount of water in the hair filler composition may be less than about 5 wt. %, based upon the total weight of the hair filler composition. In embodiments disclosed throughout this specification, the amount of water in the hair filler composition may be less than about 1 wt. %, based upon the total weight of the hair filler composition. In embodiments disclosed throughout this specification, the hair filler composition may be substantially devoid of water.

### Optional Ingredients

Optionally, the hair filler composition may include one or more additional ingredients. Optional ingredients include but are not limited to, nonionic surfactants, soaps, silicone surfactants, rheological agents, electrolytes, proteins, sunscreens, vitamins, botanical extracts, emulsifying agents, fragrances, and preservatives.

Generally, the hair filler composition may be described as a liquid at room temperature and standard pressure. The liquid hair filler composition has a viscosity that allows it to be mixed with a propellant and sprayed as an aerosol composition. The term liquid should be interpreted to include gel compositions. In embodiments disclosed throughout this specification, the hair filler composition has a viscosity under standard conditions of temperature and pressure of less than about 1000 mm²/s (cST), or from about 10 mm²/s (cST) to about 1000 mm²/s (cST).

In embodiments disclosed throughout this specification, all ingredients except the propellants may be blended and mixed with high shear mixers to form the hair filler composition, which may also be referred to as a pre-mix batch. In embodiments disclosed throughout this specification, the total solid loading of the pre-mix batch may be no more than 45 wt. %. This pre-mix may then be combined with the propellant in an aerosol dispenser adapted to store and dispense the aerosol cosmetic composition.

### Propellants

The sprayable aerosol compositions of the present invention may include one or more propellants. Propellants may be used individually or blended together. Advantageously, the selection of a propellant or blend of propellants may be used to achieve a particular spray pattern, control particle size, conform to government regulations, or for cost considerations.

Propellants may be selected from the group consisting of hydrocarbons, halocarbons, ethers, and combinations thereof. Examples of hydrocarbon propellants include pentane, n-butane, isobutane, and propane. Examples of halocarbons primarily include, but are not limited to, organochlorine compounds, organofluorine compounds such as hydrofluorocarbons, and compounds containing both fluorine and chlorine atoms. Examples of hydrofluorocarbon propellants include 1,1,1,2-tetrafluoroethane (134a) and 1,1-difluoroethane (152a). An example of an ether propellant includes dimethyl ether.

In embodiments disclosed throughout this specification, the amount of propellant may be from about 20 to about 97 wt.%, from about 30 to about 97 wt.%, from about 35 to about 96 wt. %, or from about 40 to about 95 wt. %, based upon the total weight of the aerosol cosmetic composition.

### Aerosol Dispensing System

Advantageously, the cosmetic compositions of the present invention may be dispensed as an aerosol spray. The present invention provides an aerosol dispensing system. The aerosol dispensing system is not unduly limited, and may generally be described as having a container that includes an outlet and is capable of being pressurized, a valve system, and an actuator.

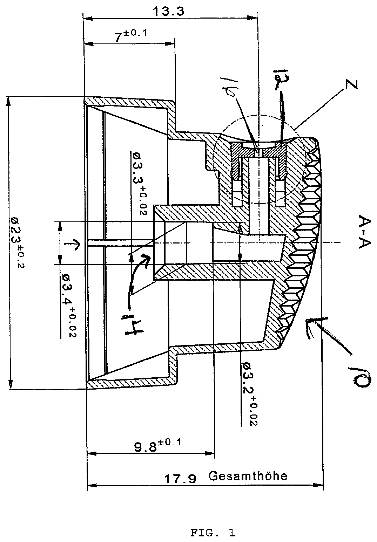

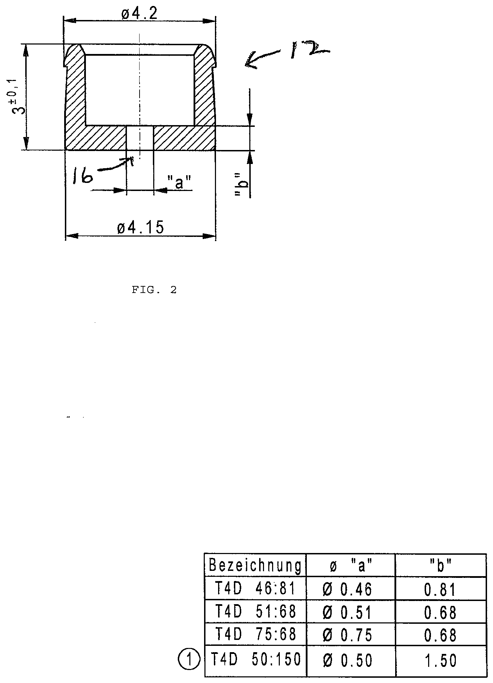

Referring now to figure 1, an actuator is shown and generally indicated by the numeral 10. Actuator 10 includes an actuator orifice insert 12 and an actuator socket 14. As shown in figure 2, orifice insert 12 defines opening 16. The diameter of opening 16 may be characterized as length "a". In embodiments disclosed throughout this specification, length a may be about 0.5mm (0.020 inches) or greater. In embodiments disclosed throughout this specification, length a may be about 0.63mm (0.025 inches) or greater.

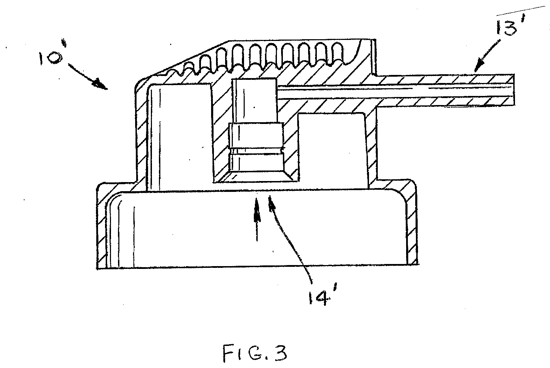

Referring to figure 3, actuator 10' is shown, and includes actuator orifice 13 and actuator socket 14'. In embodiments disclosed throughout this specification, the spray pattern may be narrowed by increasing the length of the actuator orifice 13. In embodiments disclosed throughout this specification, the actuator orifice length may be about 6.35mm (0.25 inches) or longer.

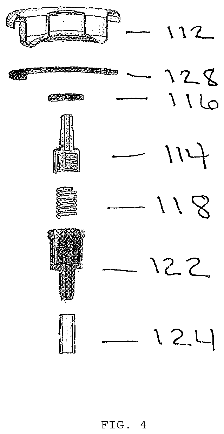

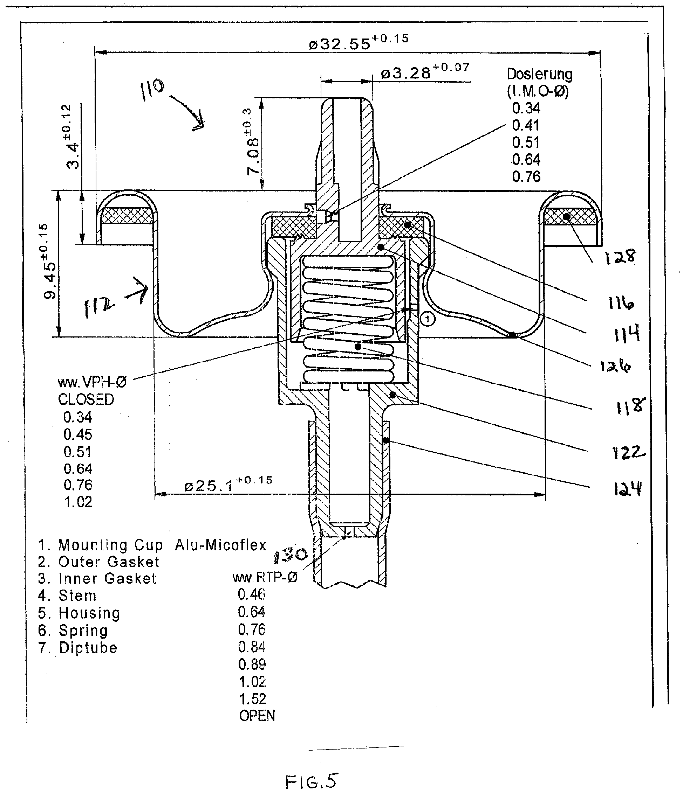

Referring now to figures 4 and 5, a valve system is shown and generally indicated by the numeral 110. Valve system 110 includes a valve housing 122. For purposes of this specification, valve housing 122 may also be referred to as valve body 122. The valve housing is surrounded by a valve cup 112. The valve cup 112 includes a mounting cup 126. Under the lip of the valve cup 112 is the mounting cup gasket 128. The valve body 122 includes an entry orifice 130 which can be mated with dip tube 124. Between the valve body 122 and the valve cup 112 is an inner gasket 116. Within the valve body is the spring 118. Above the valve body 112 is the stem 114.

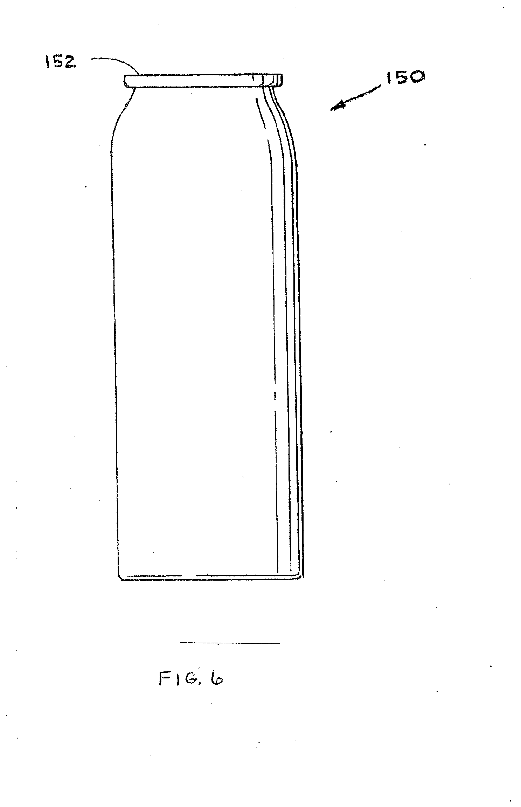

Referring now to figure 6, a can or aerosol container is shown generally indicated by the numeral 150. The can 150 includes a ridge 152, which may also be referred to as the can curl.

An aerosol can 150 can be attached, for example, to a valve system 110 by placing mounting cup 126 and gasket 128 on can curl 152 and crimping the valve cup 112. The actuator 10 can be attached, for example, to the valve system 110 by inserting the stem 114 into the actuator socket 14.

The present invention provides a method of manufacture of an aerosol hair filler, the method including the steps of combining a solvent, a plurality of fibers, a gloss enhancing oil soluble film forming polymer, and optionally one or more additional ingredients, to form a hair filler composition; placing said premix into an aerosol container 150; and then one of the following steps of: crimping said aerosol container and then filling said container with propellant; or filling said container with propellant and then crimping said aerosol container.

In embodiments disclosed throughout this specification, the propellant may be pressure filled through the valve system 110.

In embodiments disclosed throughout this specification, the propellant may be pressurized in the crimping operation. This is sometimes referred to as under the cup gassing.

In embodiments disclosed throughout this specification, the dispenser may include a container adapted to store the aerosol cosmetic composition and having an outlet adapted to dispense the aerosol cosmetic composition. The dispenser may include a valve system and actuator connected to the outlet of the container.

The valve system 110 includes a valve stem 114, a stem gasket 116, a valve body 122, a dip tube 124, a valve cup 112 , a cup gasket 128, and an entry orifice 130. The valve body may include a vapor tap orifice. Generally, a vapor tap orifice is an opening in the valve body that allows propellant for enter the valve body 122 and mix with the liquid pre-mix, which may enter the valve body 122 via the dip tube 124. The mixing of the propellant and the liquid pre-mix in the valve body 122 in this fashion produces physical agitation that may result in a smaller mean particle size of the aerosol, and/or a faster evaporation rate of the solvent in the pre-mix. The net result is a dryer spray.

Typically, an entry orifice 130 is an opening molded into the valve body 122 at the location where the dip tube enters the valve body 122. The entry orifice may be adapted to allow control of the amount of liquid pre-mix entering the valve body 122. Restricting the amount of liquid phase that enters the valve body 122 may result in more rapid expansion of the propellant within the valve body 122. This in turn may results in a smaller mean particle size of the aerosol and/or a faster evaporation rate of the solvent in the pre-mix, again resulting in a dryer spray.

Advantageously, aerosol valves may be chosen to select the spray pattern and spray rate of a product. Valve systems and spray patterns are further described in co-pending U.S. Patent Application Serial No. 13/615773.

Although there are many types of valve systems and actuators that are suitable to dispense the aerosol cosmetic compositions of the present invention, exemplary valve systems include those available from Lindal Valve Co. In embodiments disclosed throughout this specification, the valve system includes a stem valve provided by Lindal under the moniker CA39F. In embodiments disclosed throughout this specification, the actuator includes a full-cup actuator provided by Lindal under the moniker ST340A. Exemplary valve systems also include those available from Summit Valve Corporation or Precision Valve Co., with a Kosmos MiniJet actuator that is available from Precision Valve Corp. An exemplary valve for producing a wide spray is produced by Precision Valve Corp. or Lindal Valve Co. and uses a standard Kosmos type actuator.

In embodiments disclosed throughout this specification, the orifice through which the product is dispensed may have an opening having a diameter of at least about 0.63mm (0.025 inches).

Advantageously, the compositions of the present invention are suitable to be dispensed by using an aerosol dispensing system as described within, and the dispenser does not become clogged. In embodiments disclosed throughout this specification, the dispenser may be used on a daily basis, over its lifetime (i.e. until the composition is depleted and the container is empty), without clogging. More specifically, in embodiments disclosed throughout this specification, the dispenser may be actuated to dispense the aerosol sprayable cosmetic composition for a time period of from about 5 to about 10 seconds, every 24 hours for at least 14 days, and does not become clogged. In embodiments disclosed throughout this specification, the dispenser may be actuated to dispense the aerosol sprayable cosmetic composition for a time period of from about 5 to about 10 seconds, every 24 hours for at least 21 days, and does not become clogged. In embodiments disclosed throughout this specification, the dispenser is actuated to dispense the aerosol sprayable cosmetic composition for a time period of from about 5 to about 10 seconds, every 24 hours for at least 28 days, and does not become clogged.

In order to demonstrate the practice of the present invention, the following examples have been prepared and tested. The examples should not, however, be viewed as limiting the scope of the invention.

Comparison of Fiber Composition and size: Two examples of hair filler compositions were prepared according to the present invention, and differed only in the fiber composition. In Example 1, cellulose fibers were employed - specifically Cell-u-lash 40 cellulose fibers obtained from Kobo Products, Inc., having an average length of 20-25 microns. An aerosol sprayable composition according to the present invention was prepared containing said cellulose fibers, ethyl trisiloxane, trimethylsiloxysilicate, methyl trimethicone, a mixture of cosmetic hair fillers, fragrance, and a propellant mixture including hydrofluorocarbon 152a and butane. The composition was placed into a dispenser consisting of an aluminum can, a Lindal CA39F valve with a ST340A actuator having an orifice of about 0.63mm (0.025 inches), and actuated for a time period of from about 5 to about 10 seconds, every 24 hours for at least 14 days. The composition did not clog the aerosol dispenser, was easily placed into the aerosol dispenser and easily dispensed therefrom, and produced a fine, homogeneous, spray.

In Example 2, the composition as for Example 1 was used, except that Cell-u-lash 150 cellulose fibers obtained from Kobo Products, Inc., having an average length of 80-110 microns were employed instead of Cell-u-lash 40 fibers. The composition was placed into a dispenser identical to the one employed for Example 1, and was actuated according to the same schedule. Example 2 clogged the aerosol dispenser valve system after about 3 actuations, and therefore was not operable as an aerosol sprayable composition.

In Example 3, the composition as for Example 1 was used, except that nylon-based fibers were employed instead of cellulose fibers - specifically Fiberlon 93 fibers obtained from Sensient Technology, having a size of 300 microns. The composition was placed into a dispenser identical to the one employed for Example 1, and was actuated according to the same schedule. Example 3 clogged the aerosol dispenser valve system after only several actuations, and therefore was not operable as an aerosol sprayable composition.

In Example 4, the composition as for Example 1 was used, except that nylon-based fibers were employed instead of cellulose fibers - specifically Fiberlon 93 AC fibers obtained from Sensient Technology, having an average length of 500 - 1000 microns. The hair filler composition was very thick, such that it could not be poured into the aerosol container. Furthermore, this thick composition would not be suitable to pass through production lines. Therefore, Example 4 was not operable as an aerosol sprayable composition.

The present invention provides an aerosol texturizing colorant composition comprising (a) a hair filler composition that comprises: cellulose fibers; a texturizing agent; a conditioning agent; and a carrier comprising a film forming polymer, one or more colorants, and a non-aqueous solvent; and (b) a propellant.

In any of the embodiments of the colorant composition described above, composition includes from about 0.1 to about 20 wt. % of cellulose fibers, based upon the total weight of the hair filler composition.

In any of the embodiments of the colorant composition described above, the texturizing agent is a hollow microsphere complex selected from the group consisting of Silica (and) Ethylene/Methacrylate Copolymer (and) Isopropyl Titanium Triisostearate, Mica (and) Ethylene/Methacrylate Copolymer (and) Isopropyl Titanium Triisostearate, Talc (and) Ethylene/Methacrylate Copolymer (and) Isopropyl Titanium Triisostearate, Iron Oxides (and) Ethylene/Methacrylate Copolymer (and) Isopropyl Titanium Triisostearate, and combinations thereof.

In any of the embodiments of the colorant composition described above, the amount of texturing agent is from about 0.1 to about 10 wt. %, based upon the total weight of the hair filler composition.

In any of the embodiments of the colorant composition described above, the conditioning agent may be selected from the group consisting of starches and modified starches.

In any of the embodiments of the colorant composition described above, the amount of conditioning agent may be from about 3 to about 6.5 wt. %, based upon the total weight of the hair filler composition.

In any of the embodiments of the colorant composition described above, the hair filler composition may include from about 1 to about 20 wt. % oil soluble film forming polymer, based upon the total weight of the hair filler composition.

In any of the embodiments of the colorant composition described above, the colorants may include FD&C aluminum lakes, FD&C calcium lakes, D&C blues, D&C browns, D&C yellows, D&C oranges, D&C reds, D&C greens, D&C ultra marines, FD&C blues, FD&C browns, FD&C yellows, FD&C oranges, FD&C reds, FD&C greens, FD&C ultra marines, black iron oxide, aluminum powder, bronze powder, chromium, chromium hydroxide, iron oxides, titanium dioxide, zinc oxide, zinc myristate, mica, titanated mica, talc, corn starch, aluminum starch, pearlizing agents, silica, silica silyate, spherical silica, fumed silica, aluminum silicate calcium silicate, silk powder, and polyester glitter flakes. In one or more embodiments the cosmetic colorant can be surface-treated with silicones, silanes, lecithin, cationic polymer, N-lauroyl-L-lysine, amino acids, triethoxycaprylylsilane, or some combination thereof.

In any of the embodiments of the colorant composition described above, the colorant may include one or more of titanium dioxide (C.I. 77891), yellow iron oxide (C.I. 77492), red iron oxide (C.I. 77491), black iron oxide (C.I. 77499), and mixtures thereof.

In any of the embodiments of the colorant composition described above, the at least one cosmetic colorant may be present in the hair filler composition in an amount of from about 0.4 wt.% to about 25 wt.%, in other embodiments, from about 0.4 wt. % to about 22 wt. %, based upon the total weight of the hair filler composition.

In any of the embodiments of the colorant composition described above, the at least one cosmetic colorant may be present in the aerosol cosmetic composition in an amount of from about 0.01 wt.% to about 1.2 wt.%, in other embodiments, from about 0.02 wt. % to about 1.1 wt. %, based upon the total weight of the aerosol cosmetic composition.

In any of the embodiments of the colorant composition described above, the total amount of cosmetic colorant may be reduced, compared to hair colorant and root concealer compositions that do not contain fibers. In any of the embodiments of the colorant composition described above, the reduction in colorant may lead to enhanced sprayability of the compositions, while good color coverage is maintained.

In any of the embodiments of the colorant composition described above, the solvent may be a volatile silicone solvent, and the hair filler composition may include from about 45 wt.% to about 75 wt.% of the volatile silicone solvent, based upon the total weight of the hair filler composition.

The present invention also provides an aerosol dispensing system storing and adapted to dispense the aerosol sprayable colorant composition of any of the preceding embodiments.

Various modifications and alterations that do not depart from the scope of this invention will become apparent to those skilled in the art. This invention is not to be duly limited to the illustrative embodiments set forth herein.

## Claims

1. An aerosol texturizing colorant composition comprising:
(a) a hair filler composition that comprises:
from 0.1 to 20 wt. % of cellulose fibers, based upon the total weight of the hair filler composition, wherein the average length of the cellulose fibers is from 20 to 25 microns;
a texturizing agent;
a conditioning agent; and
a carrier comprising as a film forming polymer a trimethylsiloxysilicate resin, one or more colorants, and a non-aqueous solvent;
wherein the texturizing agent is a hollow microsphere complex selected from the group consisting of Silica (and) Ethylene/Methacrylate Copolymer (and) Isopropyl Titanium Triisostearate, Mica (and) Ethylene/Methacrylate Copolymer (and) Isopropyl Titanium Triisostearate, Talc (and) Ethylene/Methacrylate Copolymer (and) Isopropyl Titanium Triisostearate, Iron Oxides (and) Ethylene/Methacrylate Copolymer (and) Isopropyl Titanium Triisostearate, and combinations thereof and wherein the amount of texturing agent is from 0.1 to 10 wt. %, based upon the total weight of the hair filler composition; and
(b) a propellant;
wherein the aerosol composition is dispensable by an aerosol dispensing system for a time period of from 5-10 seconds, every 24 hours for at least 14 days, without the dispensing system becoming clogged.

2. The colorant composition of claim 1, wherein the conditioning agent is selected from the group consisting of starches and modified starches.

3. The colorant composition of any of the previous claims, wherein the amount of conditioning agent is from 3 to 6.5 wt. %, based upon the total weight of the hair filler composition.

4. The colorant composition of any of the previous claims, wherein the hair filler composition includes from 1 to 20 wt. % oil soluble film forming polymer, based upon the total weight of the hair filler composition.

5. The colorant composition of any of the previous claims, wherein the solvent is a volatile silicone solvent, and the hair filler composition includes from 45 wt.% to 75 wt.% of the volatile silicone solvent, based upon the total weight of the hair filler composition.

6. A method for creating the appearance of fuller hair by covering bald spots and areas on the scalp where hair is thin, comprising:
evenly applying the aerosol sprayable colorant composition of any of claims 1 to 5 to the scalp in a controlled pattern.

## Patentansprüche

1. Texturierungsfarbstoff-Aerosolzusammensetzung, umfassend:
(a) Haarfüller-Zusammensetzung, die umfasst:
0,1 bis 20 Gew.-% Cellulosefasern, bezogen auf das Gesamtgewicht der Haarfüller-Zusammensetzung, wobei die durchschnittliche Länge der Cellulosefasern 20 bis 25 µm beträgt;
Texturierungsmittel;
Konditionierungsmittel; und
Trägermaterial, das als filmbildendes Polymer Trimethylsiloxysilikatharz, einen oder mehrere Farbstoffe und nichtwässriges Lösungsmittel umfasst;
wobei das Texturierungsmittel ein Komplex hohler Mikrokügelchen ist, die ausgewählt sind aus der Gruppe bestehend aus Siliciumdioxid (und) Ethylen/Methacrylat-Copolymer (und) Isopropyltitantriisostearat, Glimmer (und) Ethylen/Methacrylat-Copolymer (und) Isopropyltitantriisostearat, Talk (und) Ethylen/Methacrylat-Copolymer (und) Isopropyltitantriisostearat, Eisenoxide (und) Ethylen/Methacrylat-Copolymer (und) Isopropyltitantriisostearat Triisostearat und Kombinationen davon, wobei die Menge an Texturierungsmittel, bezogen auf das Gesamtgewicht der Haarfüller-Zusammensetzung, 0,1 bis 10 Gew.-% beträgt; und
(b) Treibmittel;
wobei die Aerosolzusammensetzung durch ein Aerosolabgabesystem für einen Zeitraum von 5 bis 10 Sekunden alle 24 Stunden für mindestens 14 Tage abgegeben werden kann, ohne dass das Abgabesystem verstopft.

2. Texturierungsfarbstoff-Aerosolzusammensetzung nach Anspruch 1, wobei das Konditionierungsmittel ausgewählt ist aus der Gruppe bestehend aus Stärken und modifizierten Stärken.

3. Texturierungsfarbstoff-Aerosolzusammensetzung nach einem der vorhergehenden Ansprüche, wobei die Menge an Konditionierungsmittel, bezogen auf das Gesamtgewicht der Haarfüller-Zusammensetzung, 3 bis 6,5 Gew.-% beträgt.

4. Texturierungsfarbstoff-Aerosolzusammensetzung nach einem der vorhergehenden Ansprüche, wobei die Haarfüller-Zusammensetzung 1 bis 20 Gew.-% eines öllöslichen filmbildenden Polymers, bezogen auf das Gesamtgewicht der Haarfüller-Zusammensetzung, enthält.

5. Haarfüller-Zusammensetzung nach einem der vorhergehenden Ansprüche, wobei das Lösungsmittel ein flüchtiges Silikonlösungsmittel ist und die Haarfüller-Zusammensetzung 45 Gew.-% bis 75 Gew.-% des flüchtigen Silikonlösungsmittels, bezogen auf das Gesamtgewicht der Haarfüller-Zusammensetzung, enthält.

6. Verfahren zum Erzeugen des Eindrucks von volleren Haaren durch Abdecken von kahlen Stellen und Bereichen auf der Kopfhaut, wo das Haar dünn ist, umfassend:
gleichmäßiges Auftragen der Haarfüller-Zusammensetzung gemäß einem der Ansprüche 1 bis 5 auf die Kopfhaut in einem kontrollierten Muster.

## Revendications

1. Composition d'agents colorants texturisants sous forme d'aérosol comprenant:
(a) une composition de remplissage capillaire qui comprend:
- de 10,1 à 20 wt % de fibres de cellulose, sur la base du poids total de la composition de remplissage capillaire, la longueur moyenne des fibres de cellulose étant comprise entre 20 et 25 microns
- un agent texturisant;
- un agent conditionnant; et
- un porteur comprenant, comme résine filmogène, un résine de triméthylsiloxysilicate, un ou plusieurs colorants, et un solvant non aqueux;
dans laquelle l'agent texturisant est un complexe de microbilles creuses sélectionné parmi le groupe consistant en Silice (et) Copolymère Éthylène/Méthacrylate (et) Triisostéate d'Isopropyl Titanium, Mica (et) Copolymère Éthylène/Méthacrylate (et) Triisostéate d'Isopropyl Titanium, Talc (et) Copolymère Éthylène/Méthacrylate (et) Triisostéate d'Isopropyl Titanium, Oxydes de fer (et) Copolymère Éthylène/Méthacrylate (et) Triisostéate d'Isopropyl Titanium, et leurs associations, et dans lequel la quantité d'agent texturant est d'environ 0,1 à environ 10 wt %, sur la base du poids total de la composition de remplissage capillaire; et
(b) un propulseur;
dans lequel la composition d'aérosol est dispensée par un système de distribution d'aérosol pendant une période de 5 à 10 secondes, tous les 24 heures, pendant au moins 14 jours, sans que le système de distribution ne se bouche.

2. Composition colorante selon la revendication 1, dans laquelle l'agent conditionnant est sélectionné parmi le groupe consistant en amidons et amidons modifiés.

3. Composition colorante selon l'une quelconque des revendications précédentes, dans laquelle la quantité d'agent conditionnant est comprise entre 3 et 6,5 wt %, sur la base du poids total de la composition de remplissage capillaire.

4. Composition colorante selon l'une quelconque des revendications précédentes, dans laquelle la composition de remplissage capillaire comprend de 1 à 20 wt % de polymère filmogène soluble dans l'huile, sur la base du poids total de la composition de remplissage capillaire.

5. Composition colorante selon l'une quelconque des revendications précédentes, dans laquelle le solvant est un solvant silicone volatil, et la composition de remplissage capillaire comprend de 45 wt % à 75 wt % de solvant silicone volatil, sur la base du poids total de la composition de remplissage capillaire.

6. Procédé pour donner l'apparence d'un cheveux plus volumineux en couvrant les zones dégarnies et les zones du cuir chevelu où les cheveux sont fins, comprenant l'application uniforme de la composition colorante aérosol sprayable selon l'une quelconque des revendications 1 à 5 sur le cuir chevelu selon un motif contrôlé.
